# EUROPEAN PATENT APPLICATION

(11) **EP 1 818 415 A1**
(43) Date of publication of application: **15.08.2007**
(21) Application number: 06290237.4
(22) Date of filing: 10.02.2006
(51) Int. Cl.: C12Q 1/68

(54) **Method of molecular evolution with synthetic DNA**

(71) Applicant: Sarl Protein Biosensor, 31400 Toulouse (FR)
(72) Inventor: Fournier, Didier, 31000 Toulouse (FR); Demange, Pascal, 31400 Toulouse (FR)
(74) Representative: Ahner, Francis

(57) **Abstract**

The present invention relates a method to obtain a synthetic gene devoid of frameshift mutations. It is a qualitative and semi-quantitative method for producing proteins and polypeptides starting from synthetic DNA sequences which are co-expressed in frame with a reporter protein in transformed micro-organisms. This allows selection of expressed proteins and discard of frame shift and non-sense mutations. The invention also relates to a new molecular evolution technique based on random mutations occurring during DNA synthesis. This method takes advantages of the polymorphism existing during DNA synthesis to promote the construction of a library leading to mutated proteins with improved properties and/or modified activities. It permits to simultaneously perform multi-site directed mutagenesis and random mutagenesis for molecular evolution.

SEQUENCE LISTING

<110> PROTEIN BIOSENSOR

<120> Method of molecular evolution with synthetic dna

<130> D24180

<160> 14

<170> PatentIn version 3.3


<210> 1
<211> 234
<212> DNA
<213> artificial sequence

<220>
<223> Sequence of the promoter, polylinker, and GFP fusion
      region of the pGT vector

<400> 1
ccgtattacc gcctttgagt gagctgatac cgctcgccgc agccgaacga ccgagcgcag      60
cgagtcagtg agcgaggaag cgagctgttg acaattaatc aggctcgtat aatgtgtggt     120
gaattcctgc agcccggggg atccaagctt agcaagggcg aggagctgtt caccgacgag     180
ctggtgccat gaggtaccca attcgcccta tagtgagtcg tattacgcgc gctc           234


<210> 2
<211> 217
<212> DNA
<213> artificial sequence

<220>
<223> Sequence of the promoter, polylinker, and GFP fusion
      region of the pTRAG vector

<400> 2
ctcgccgcag ccgaacgacc gagcgcagcg agtcagtgag cgaggaagcg agctgttgac      60
aattaatcag gctcgtataa tgtgtggtga attcgtgagc ggataacaag gagatatacc     120
atgacccatc accatcacca tcaccgcccg ggggatccaa gcttagcaag ggcgaggagc     180
tgttcaccga cgagctggtg ccatgaggta cccaatt                              217


<210> 3
<211> 248
<212> DNA
<213> artificial sequence

<220>
<223> Sequence of the promoter, polylinker, and GFP fusion
      region of the pETG vector

<400> 3
caacgctgcc cggagatctc gatcccgcga aattaatacg actcactata gggagaccac      60
aacggtttcc ctctagaaat aattttgttt aactttaaga aggagatata catatggcta     120
gcatgactgg tggacagcaa atgggtcggg attcaagctt agcaagggcg aggagctgtt     180
caccgacgag ctggtgccat gaggtaccga gctcggatcc actagtaacg gccgccagtg     240
tgctggaa                                                              248

<210> 4
<211> 323
<212> DNA
<213> artificial sequence

<220>
<223> Sequence of the promoter, polylinker, and GFP fusion
      region of the pcocoG vector


<400> 4
ctcgatcccg cgaaattaat acgactcact ataggggaat tgtgagcgga taacaattcc      60
cctctagaaa taattttgtt taactttaag aaggagatat aagcatgcat caccatcacc     120
atcacggtgc tagcaaagaa ccgctgctgc gaaatttgaa cgccagcaca tggactcgcc     180
acgccaactg tgatgacgac gacaagagcc cgggcgatcg catcgatgcc aggcgcgcca     240
cgtgcgtacg gtttcgaagc ttagcaaggg cgaggagctg ttcaccgacg agctggtgcc     300
atgaggtacc caattcgccc tat                                             323


<210> 5
<211> 13
<212> PRT
<213> artificial sequence

<220>
<223> artificial sequence

<400> 5

Ser Lys Gly Glu Glu Leu Phe Thr Asp Glu Leu Val Pro
1               5                   10


<210> 6
<211> 46
<212> DNA
<213> artificial sequence

<220>
<223> artificial sequence

<400> 6
agcggagctg ttgacaatta atcatcggct cgtataatgt gtggag                    46


<210> 7
<211> 46
<212> DNA
<213> artificial sequence

<220>
<223> artificial sequence

<400> 7
ctcgacaact gttaattagt agccgagcat attacacacc acttaa                    46


<210> 8
<211> 37
<212> DNA

<213> artificial sequence

<220>
<223> sequence obtained by insertion of the tac promoter
      upstream the GFP5 coding sequence

<400> 8
agctgttgaa aattaatcat cggctcgtat aatgtgt                                37


<210> 9
<211> 39
<212> DNA
<213> artificial sequence

<220>
<223> sequence obtained by insertion of the tac promoter
      upstream the GFP6 coding sequence


<400> 9
agctgttgaa attaatcatc ggctcgtata atgtgtgga                              39


<210> 10
<211> 38
<212> DNA
<213> artificial sequence

<220>
<223> sequence obtained by insertion of the tac promoter
      upstream the GFP14 coding sequence


<400> 10
agctgttgac aattaatcag gctcgtataa tgtgtggt                               38


<210> 11
<211> 257
<212> DNA
<213> artificial sequence

<220>
<223> Synthetic genes without screening

<400> 11
atttccatgg cgattgcgct gggctatacc aattctggtc tgaatccggt attatatgcg      60
tttctggatg aaaattttaa acgtgcgttt cgtgaatttt gcattccgac cagctctaat     120
attgaacagc agaatagcac ccgcattcgc cagaataccc gtgatcatcc gagcaccgcg     180
aataccgtgg atcgcaccaa tcatcagtta gaaaatttag aagcggaaac cgcgccgtta     240
ccgtaatctc gagtttt                                                    257


<210> 12
<211> 79
<212> PRT
<213> artificial sequence

<220>
<223> peptidic sequence corresponding to Seq. ID N°11

<400> 12

Met Ala Ile Ala Leu Gly Tyr Thr Asn Ser Gly Leu Asn Pro Val Leu
1               5                   10                  15
Tyr Ala Phe Leu Asp Glu Asn Phe Lys Arg Ala Phe Arg Glu Phe Cys
            20                  25                  30
Ile Pro Thr Ser Ser Asn Ile Glu Gln Gln Asn Ser Thr Arg Ile Arg
        35                  40                  45
Gln Asn Thr Arg Asp His Pro Ser Thr Ala Asn Thr Val Asp Arg Thr
    50                  55                  60
Asn His Gln Leu Glu Asn Leu Glu Ala Glu Thr Ala Pro Leu Pro
65                  70                  75


<210> 13
<211> 257
<212> DNA
<213> artificial sequence

<220>
<223> synthetic genes screening by GFP fusion

<400> 13
atttccatgg cgattgcgct gggctatacc aattctggtc tgaatccggt attatatgcg      60
tttctggatg aaaattttaa acgtgcgttt cgtgaatttt gcattccgac cagctctaat     120
attgaacagc agaatagcac ccgcattcgc cagaataccc gtgatcatcc gagcaccgcg     180
aataccgtgg atcgcaccaa tcatcagtta gaaaatttag aagcggaaac cgcgccgtta     240
ccgtaatctc gagtttt                                                    257


<210> 14
<211> 79
<212> PRT
<213> artificial sequence

<220>
<223> peptidic sequence corresponding to Seq ID N°8

<400> 14

Met Ala Ile Ala Leu Gly Tyr Thr Asn Ser Gly Leu Asn Pro Val Leu
1               5                   10                  15
Tyr Ala Phe Leu Asp Glu Asn Phe Lys Arg Ala Phe Arg Glu Phe Cys
            20                  25                  30
Ile Pro Thr Ser Ser Asn Ile Glu Gln Gln Asn Ser Thr Arg Ile Arg
        35                  40                  45
Gln Asn Thr Arg Asp His Pro Ser Thr Ala Asn Thr Val Asp Arg Thr
    50                  55                  60
Asn His Gln Leu Glu Asn Leu Glu Ala Glu Thr Ala Pro Leu Pro
65                  70                  75

## Description

The present invention relates a method to obtain a synthetic gene devoid of frameshift mutations. It is a qualitative and semi-quantitative method for producing proteins and polypeptides starting from synthetic DNA sequences which are co-expressed in frame with a reporter protein in transformed micro-organisms. This allows selection of expressed proteins and discard of frame shift and non-sense mutations. The invention also relates to a new molecular evolution technique based on random mutations occurring during DNA synthesis. This method takes advantages of the polymorphism existing during DNA synthesis to promote the construction of a library leading to mutated proteins with improved properties and/or modified activities. It permits to simultaneously perform multi-site directed mutagenesis and random mutagenesis for molecular evolution.

### Background of the invention

Synthetic genes made from oligonucleotide assembly are used from the late of 70's (1-3) and today several methods are currently available (4-6). This technique draws considerable attention in protein engineering when the desired DNA sequence is not available or is difficult to clone (7). This technique is also of interest when the sequence needs to be optimized for a suitable heterologous expression. Optimization may be obtained by choosing appropriate codon (8-12), by improving the initiation of translation (9) and by elimination of polyadenylation signals (13, 14), false priming events (10), RNA strong secondary structures (10) sequences that contribute to RNA instability (15) or signal of post-translational modifications (9). Then, the corrections that are needed to adapt the sequence are often too numerous to be considered by site directed mutagenesis and synthetic gene appeared to be the best solution. In addition, synthesizing a gene permit to add many unique restriction sites to facilitate subsequent cloning steps (10, 12, 16). However, assembly of oligonucleotides usually results in frequent DNA sequence errors (17-20). The starting material of a synthetic gene is a set of several long oligonucleotides which sequences are not accurate enough. Despite the excellent yield of coupling reaction between two nucleotides (yield ~ 99.99%), the length and the number of oligonucleotides covering all together more that 1 kbase will always lead to byproducts containing internal deletions or point mutations. Moreover, subsequent PCR used to amplify the full-length sequence may introduce additional mutations to the final DNA fragment. Consequently, clones obtained by synthetic assembly of oligonucleotides have often to be screened to find the correct one or have to be repaired (11, 12, 14, 17, 20-29).
One way to limit deletions is to previously purify oligonucleotides by electrophoresis (30). This purification according to the size allows to select molecules devoid of deletion and its efficiency decrease with the oligonucleotide length leading to the use of oligonucleotide shorter than 60 nucleotides (19). Another way is to build small 500 to 800-bp "synthons", to verify their sequences and finally to join them to obtain longer sequences (19, 31). Another possibility is to remove error-containing sequences by selective digestion using mismatch repair proteins or by binding on MutS (26, 27, 32).

But, these additional experiments are time and cost consuming either for the DNA manufacturers or for the end-users.

Consequently, there is an unmet need for alternative solutions that would avoid the above mentionned problems.

In connection with the invention, we tested another strategy using a reporter protein, which is expressed only when the synthetic protein is entire. Here, we used the fluorescence properties of the GFP as a biomarker to select clones expressing the synthetic genes in frame with GFP. Indeed, we observed that there are 3.5 nucleotide deletions per kb when using commercial oligonucleotides. Besides that are about 3 mutations per kb some of which may be non-sense. We thus propose a strategy to overcome this problem by cloning the synthetic gene upstream with a reporter gene so that the insert promotes its expression by providing translation initiation motives in the right frame. Selecting of GFP expressing micro-organisms allowed to identify and remove synthetic sequences which harbor a shift in the reading frame.

In addition, among the 3 non-neutral mutations per kb some are of interest and allowed us to select clones with mutations improving or modifying the activity of protein of interest. Thus, the method of the invention turns the problem of errors found in synthetic genes as an advantage by providing the polymorphism useful for molecular evolution to lead to improved polypeptides and proteins useful in therapy or enzyme as new improved biocatalysts.

### Description

Therefore, in a first embodiment, the invention is directed to a method of producing mutated proteins and polypeptides comprising the steps consisting of:
a) inserting synthetic DNA fragments within a construct comprising a promoter allowing expression in micro-organisms and a reporter gene sequence;
b) transforming said micro-organisms with the construct obtained in step a);
c) culturing transformed micro-organisms of step b) and observing the expression of the reporter gene, wherein the expression of the reporter gene is indicative of expression of said synthetic DNA fragment, and wherein no expression of the reporter gene is indicative of frameshift and non-sense mutations, and
d) selecting transformed micro-organisms expressing the reporter protein,
e) optionally, testing the activity and/or properties of the protein or polypeptide expressed from synthetic DNA sequences selected in step d).

In step d), frameshift and non-sense mutations are eliminated by screening miro-organism colonnies expressing the reporter gene. This allows to benefit from our observation of 3 mutations per kb in synthetic DNA while specifically removing mutations leading to loss of expression of the protein. Basically, this method can be applied to improve existing proteins, therapeutic polypeptides and enzymes currently developped in bioprocessing. By improvement, it will be understood that one aim is to provide proteins and polypeptides with longer half-life, stability, increased productivity, enhanced solubility, or increased activity, modified selectivity, such as increasing or decreasing subtrate spectrum, or new functionnality.

The above method can also be applicable to DNA manufacturers to provide customers with quality improved end-products. Indeed, it non only eliminates in step d) frameshift and non-sense mutations but it also enable to deplete detrimental sequences containing mutations resulting in unproper folding of the protein that aggregates to the reporter proteins impeding proper folding of the reporter protein. Here, so far as DNA manufacturers are concerned, step d) is optional.

By synthetic DNA sequence, it will be understood any sequences including complete and partial genes and coding sequences or parts thereof synthesized by chemical reactions refered as DNA synthesis commonly known by the skilled in the art (Caruthers, M.H. (1985) Gene synthesis machines: DNA chemistry and its uses. Science, 230, 281-285). Chemical DNA synthesis is a cyclical process that assembles a chain of nucleotides from the 3'-end to the 5'-end. The 3' nucleoside is covalently attached to a solid support and successive nucleotide monomers are added one by one through a cycle of four chemical reactions, one common unit being the Perkin Elmer/Applied Biosystems (PE/AB) 394 DNA synthesizer in academic settings while the PE/AB 391 model is used by commercial manufacturers.

In the above method errors occuring during synthetic DNA fragment synthesis provide synthetic gene polymorphism. In one specific embodiment, the method may further be combined with multi-site directed mutagenesis. Thus, in step c), a library of mutants polypeptide or proteins for molecular evolution is provided.

Among reporter proteins, it is advantageous to use auto-fluorescent proteins, such as GFP (Green Fluorescence Protein), enhanced GFP (EGFP), YFP, BFP, RFP, as well as any variants thereof. Reporter proteins may also include dectectable enzymes by fluorescence, phosphorescence, or histochimistry means. We can cite for example β-galactosidase, alcaline phosphatase, luciferase, or chloramphenicol-acetyl-transferase (CAT).

Preferably, the GFP or variants thereof is chosen as reporter protein.

Herein, the reporter protein acts as a qualitative (folding and/or solubility) and semi-quantitative (expression rate of the protein) reporter. The difference of fluorescence observed between the different colonnies allows a semi-quantitative approach to select the most expressed clone (Ref Wado).

In the above method, it is possible to introduce into the construct a specific part of a coding sequence that one wish to mutate. This part may correspond for example to the coding sequence of the active site of an enzyme. It is thereof within our scope to introduce in step a) synthetic DNA fragments ranging from 30 bases to 2000 bases, for example 30, 60, 90 bases or 300 bases ; corresponding to a region of interest to mutate in the construct. Here, while mutations are ramdon there are located in a particular region responsible for the activity, selectivity, stability or solubility of the protein to modify.

Constructs can be suitable expression vectors - specific non limitating embodiments are displayed in example 2. The synthetic DNA may be amplified before cloning into the vector. To this end, a restriction site can be introduced within the primers used for PCR amplification allowing ligation of the amplified products in the vector. Therefore, the invention is directed to the above method wherein additional sequences are added at the extremities of synthetic sequences, said additional sequences comprising one or several restriction sites allowing cloning into said vector and sequences complementary to primers used for PCR amplification.

Among prefered micro-organisms, it is possible to perform the invention with prokaryote micro-organisms, such as E. Coli, eukaryote micro-organisms such as yeast (Pichia, Saccharomyces) or other eukaryote cells..

Once the above selection is terminated, the synthetic gene of interest can be cloned and used in other expression vector for testing activity. Alternatively, it is possible to simply eliminate the GFP coding sequence by digestion with restriction enzymes at both sides of the tag and ligation. Further details in given in example 4 below.

In a second embodiment, the invention is aimed at a kit for performing synthesis and expression of synthetic genes ::
- for in vitro synthesis of genes synthesis, an enzyme necessary for the phosphorylation, an enzyme for the ligation (ex: T4 DNA ligase) and for the PCR (Taq DNA polymerase and/or the oligo) and the two primers used for amplification and ligation in expression vector.
- For expression, the vector with the reporter protein and the competent cells E. Coli.

The kit according to the invention provides an efficient method for screening and expressing synthetics genes. It is suitable for making a molecular evolution of proteins to improve their production, stability, selectivity, solubility, and/or increased activity.

This kit will provide users the currents tools to construct a library of synthetic genes leading to mutated proteins (improved production, stability, selectivity, solubility, novel properties...). From the synthetic sequences of the protein, the user should order oligonucleotides covering the coding sequence. Extremities of said synthetic sequences are first enlarged (phosporylation and ligation) with additional sequences described in the protocol of the kit to allow cloning into the expression vector. These two primers may use for PCR amplification and introduce unique restriction sites and the RBS sequence to first facitate the ligation and secondary to express the mutated sequences of interest. Thus, the kit may contains enzymes for incorporating synthetics genes in a screening and expression vectors including a restriction enzyme.

### Example:

Step 1: Phosphorylation of 5' extremities : The kit contains the enzyme to phosphorylate the 5' extremities, the substrate and the buffer.
Step 2: Ligation : The kit contains the enzyme for ligation and its buffer.
Step 3 : PCR amplification with primers corresponding to sequence 1 and 2 both reverse:
   The kit contains the enzyme for PCR, as the amorces for amplification and the buffer.
Step 4 : Digestion by restriction enzyme EcoRI and HindIII: The kit contains the restriction enzymes for digestion and their buffers
Step 5 : Insertion in pTG cut by Eco RI / Hind III and transformes in E. Coli:
   The kit may contain the pTG vector (with reporter fusion protein for screening), the restriction enzymes, the competent E.Coli.

Starting with this library, several DNA fragments may be replaced to increase the polymorphism or to perform site directed mutagenesis.

It will be referred to the figure legends in the following detailed description.

### Figure legends

**Figure 1:** Annotated DNA sequence of the promoter, polylinker, and GFP fusion region of the 4 vectors
**Figure 2:** Variations in sequences obtained by insertion the tac promoter upstream the GFP coding sequence, and selection of colonies stable for GFP production.
   Sequences, which are different from the inserted promoter, are underlined and deletion are symbolized by a dash. Figure legend text.
**Figure 3:** Comparison of sequences obtained by construction of a small synthetic gene Clones A1 to A6 were obtained without selection and clones B1 to B6 were obtained with selection for the absence of frameshift, by cloning in pTG.
**Fig. 4:** Genotyping of colonies according to the expression of the GFP fused protein. Two groups of colonies were selected after an in vitro mutagenesis experiment according to their fluorescence. Presence of an amplification product indicates the genotype. The most fluorescent colonies encode a proline at position 289 of PP1α while the less fluorescent colonies encode a leucine. Thus proline favorizes production of a soluble protein.

### Example 1 : Quantification of synthetic genes errors such as deletions and mutations

### 1.1 Materials and Methods

Materials. Commercially available oligonucleotides were obtained from Sigma Genosys or Eurobio. They were deprotected and desalted but not size purified on gel. All oligonucleotides were designed to adjust codon usage using the web site http://www.entelechon.com/. All the reagents used were biology molecular grade. PET17b and pETcoco were from Novagen.

Gene synthesis and expression. We tested three methods to synthesize genes from a set of oligonucleotides. The first one, called "assembly PCR", consists to perform a PCR reaction with partially overlapping oligonucleotides, to fill non overlapping portions with a polymerase and to amplify the full length fragment using a PCR reaction (5, 33). A second method consists in the stepwise elongation of sequence by PCR. The gene is elongated from the middle by using primers which hybridize only in their 3' ends in each PCR reaction (34, 35). The third method appeared to be most efficient in our hands: gene synthesis and assembly were obtained following a ligation of preformed duplexes of phosphorylated overlapping oligonucleotides using ligase chain reaction to increase the probability to obtain the desired full length product which was amplified by PCR to facilitate subsequent cloning (36). Briefly 100 +/- 20 bp oligonucleotides were designed for both strands based on protein sequences previously published. The oligonucleotides were designed to have an overlap of at least 20 mers. Oligonucleotides were phosphorylated using the T4 DNA polynucleotide kinase. They were mixed and submitted to 25 cycles of ligase chain reaction using Taq DNA ligase. The ligation product was used as template for a PCR amplification using optimal hybridization temperature. Amplified fragments were purified by gel electrophoresis and inserted in vectors using unique restriction sites present in the PCR primers and transformation of bacteria (MC1061 or BL21 DE3) was made by electroporation. Cultures were grown at 37°C in rich medium containing appropriate antibiotics for selection. UV illuminating at 254 nm screened positive GFP clones.

Genotype determination. Identification of mutation was done by sequencing or by PASA (PCR amplification of specific Allele) according to Sommer et al (37). This technique relies to the fact that amplification occurs only when the 3'end of the primer matches to the DNA target. Two PCRs are performed, one with a primer specific for the wild type and one with a primer specific for the mutant. Thus, depending on the presence of an amplification product with one of both primers, it is possible to determine the genotype at the targeted nucleotide.

### 1.2 Results

Estimation of error frequency in synthetic gene construction. We constructed five different synthetic genes coding for various proteins in term of function and origin: the rabbit protein phosphatases 1α and 2A (Phosphatase 1α, 1100 bp, X07798; Phosphatase 2A, 1050 bp, P67777), a protein phosphatase from the methanogenic archaeon TM1 (PP1-arch 2, 900 bp, U96772) to construct biosensors detecting algal toxins; equinatoxin V, a haemolysin from Actinia equina (500 bp, U51900); the C terminal part of a G coupled receptor, human MOR-1 (250 bp, P35372). These genes were placed downstream from the T7 RNA polymerase promoter and the constructs were transformed in an E. coli strain free from this specific polymerase to avoid any translation of the synthetic gene. This system was chosen to avoid any bias due to the expression of a toxic protein, which would be detrimental for the host because in that case, only clones with a mutation inactivating the protein would grow. We observed 3.5 one-basepair deletions every 1000 bases. Each of them causes frameshift which usually result to an early termination of the protein. In addition we found 0.3 nonsense mutation giving stop codon (Table 1). Combined, this gives 3.8 mutations for 1000 bases which results to a truncated protein. This result is higher than the 1 non sense mutation per kb already reported (18, 26) and may originate from the differential reliability of oligonucleotide synthesis and the method used to assemble oligonucleotides (19). Taking in account 3.8 deletion and non sens mutations per kb, and according to the Poisson law, 1/33 and 1/ 2000 clones will produce a non truncated protein for a 1 and 2 kb gene respectively.

### Example 2: GFP fusion to screen clones without framshift mutations.

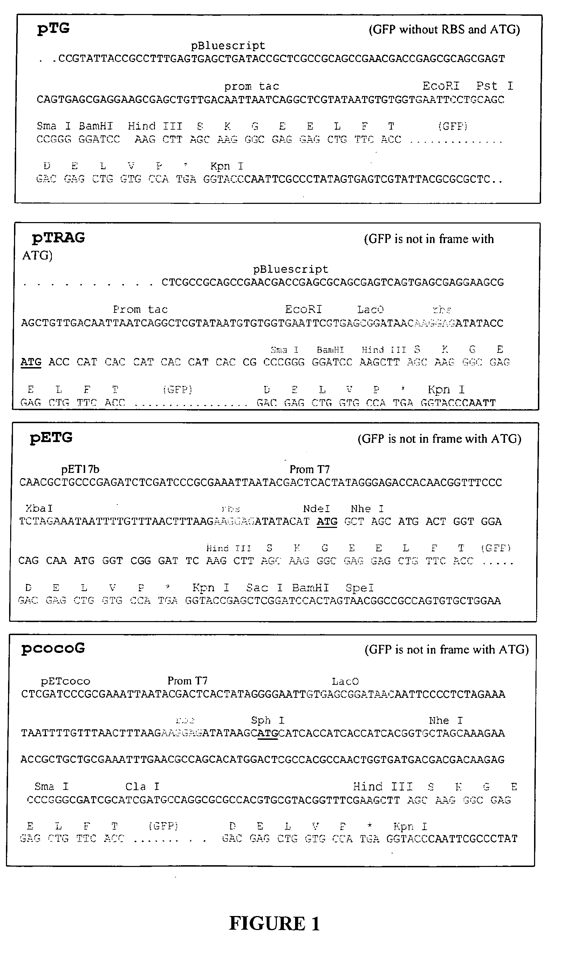

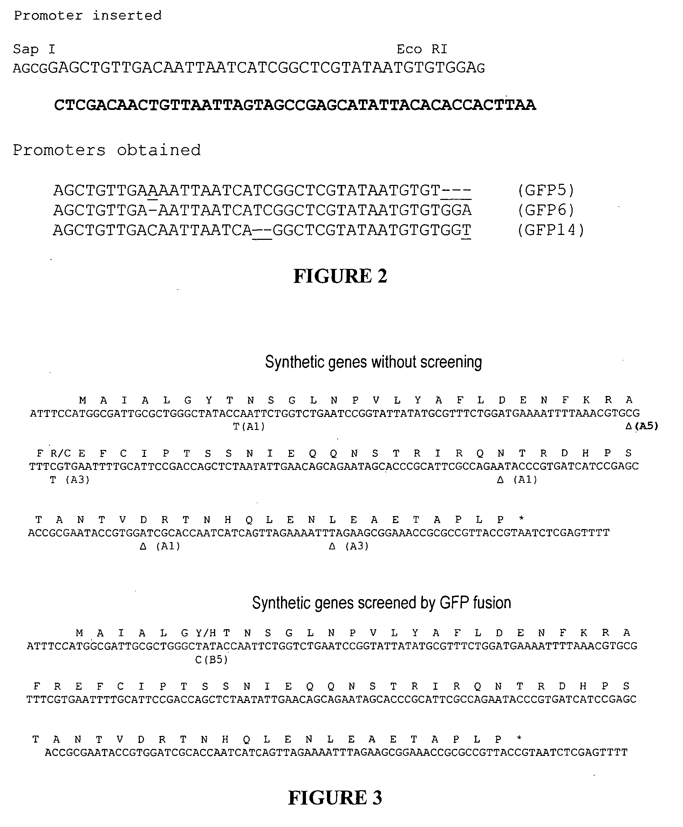

A first vector with a truncated GFP (pTG, Fig. 1) was constructed. eGFP was cloned in the EcoRI/KpnI sites of pBluescript SK-, a high copy number plasmid easy to manipulate. A tac promoter was added upstream the GFP, between the sites SapI and EcoRI in replacement of lacUV5 promoter, in order to increase the reporter gene transcription (38). This insertion removed the remaining multiple cloning site region of pSK-. Green colonies, which remained stained after several subcultures, were selected. They all contained a modified tac promoter, suggesting that overtranscription of GFP was detrimental for the host (Fig 2). A new polylinker (EcoRI/PstI/SmaI/BamHI/HindIII) was then added between the tac promoter and the GFP replacing the RBS and ATG, which have to be brought by the synthetic gene. We cloned a synthetic gene both in pTG and in pBluescript. The recombinant vectors were then transformed in E.coli and 6 clones were selected per vector. Figure 3 shows a comparison of the sequences of these 12 clones. Without screening we found 4 deletions in the 6 clones sequenced whereas no deletion was found when GFP selection was performed.

Oligonucleotide errors as variability for molecular evolution. Analysis of synthetic clones also revealed missense mutations originating either from oligonucleotide synthesis or from PCR step. The observed error number was 3 per kb (Table 1).

**Table 1: Mutations found in synthetic genes**

| Protein | Missense mutation (non neutral) | Mutation (Stop codon) | deletion | bp sequenced |
|---|---|---|---|---|
| MOR-1 C-terminal | 1 | 0 | 4 | 1452 |
| Protein phosphatase 1 | 14 | 3 | 8 | 3250 |
| PP1-arch 2 | 3 2 | | 4 | 1789 |
| Protein phosphatase 2 | 18 | 0 | 30 | 6270 |
| Equinatoxin | 10 | 0 | 9 | 3000 |
| | | | | |
| Sum | 46 | 5 | 55 | 15761 |
| mutation/1000 bases | 2.9 | 0.3 | 3.5 | |

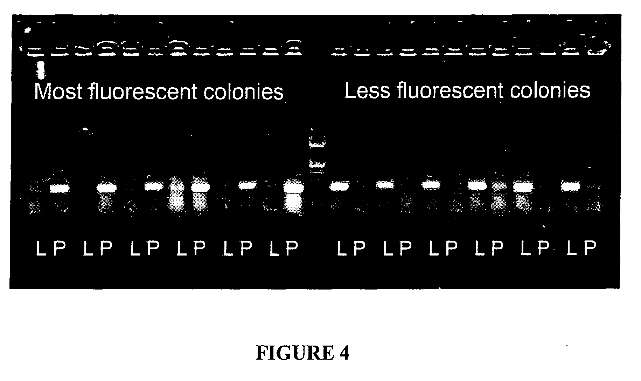

Thus, in example illustrated in Figure 3, a cystein replaced an arginine in position 29 of clone A3 (without screening) and a histidine replaced a tyrosine in position 7 of clone B5 (with screening). As expected, GFP screening did not allow eliminating missense mutations unless those which impair protein translation or folding. This strategy of using GFP translation at the C-terminus of the expected protein can be also used as a "folding reporter strategy" used to screen soluble mutants (39-41) relying on the following law: if the protein folds into a native conformation, the reporter protein will be active. Thus, if a mutation increases both protein production and solubility, it will produce a colony with increased fluorescence and will be selected. Consequently it is possible to exploit errors in synthesis as a source of molecular evolution, to screen proteins, which are produced in sufficient amount and correctly folded. This happens when we constructed the gene coding for protein phosphatase 1. One mutation L289P was present in a clone expressing GFP. To verify the importance of this mutation for the folding or production of the protein, mutagenesis was performed on this clone to revert the mutation. As usual, two kinds of clone were present in petri dishes, some wild types with a Leu and some mutated with a proline. Six clones among the most fluorescent and six clones among the less fluorescent were selected and these 12 clones were analyzed for the presence of Leu or Pro at position 289 by PASA. Result is shown on Fig. 4. The leucine to proline mutation increased the production of a soluble protein since the most fluorescent colonies have a proline at position 289 while all less fluorescent colonies had a leucine. Thus, the mutation originating from oligonucleotide error may be used as source of variability for molecular evolution.

Variability may be also increased by introducing degenerated oligonucleotides during the gene synthesis so that the synthetic shuffling resulting from the ligation and PCR steps produces a library (42) which can be screened by the GFP expression. As example, when we expressed the N terminal part of a G coupled receptor, human MOR-1, codon for Cys 13 was degenerated and coded either for cysteine or for serine. Screening of the most fluorescent colonies selected serine, its involvement in the solubility of this protein was verified by retro-mutagenesis as before.

If needed, variability may be further increased by replacing the PCR step by an error-prone PCR that will introduce random mutations in the sequence (43), however 3 mutations per kb may be considered as enough for molecular evolution to do not accumulate mutation with adversed effects.

Limitations. GFP permits to screen clones without deletion if the protein is translated and soluble. If not, this method may permit to find a clone with a mutation which increases production of a soluble protein but several false positives may complicate the screening. It occured for several genes, following transformation in pTG (Fig 1), we found several fluorescent clones. Restriction map of these clones showed that they corresponded to truncated inserts with only the first part of the gene, with the ribosome binding site and the initiation codon. Then, a first verification of lengh of insert is necessary. However, it is not sufficient. In some cases, we found deletion causing stop codons and internal initiations permitting the translation of GFP. Thus screening for GFP production may select either truncated clones with only the first part of the expected insert or clones with an internal initiation.

### Example 3 : GFP reporter vectors for toxic proteins.

pTG is a vector without any regulation system and is convenient for most of proteins. However, toxic protein expression would not be possible and no green colonies would appear except when a mutation would disrupt the protein activity without altering its folding. To solve this problem three other vectors were constructed with different levels of protein production regulation (Table 2, Fig. 3).
In pTRAG, a lacO sequence was added downstream pTG tac promoter to permit a regulation with IPTG. In addition a RBS, ATG, and a 6 histidines coding sequence were added in front of the GFP coding sequence. However, in this vector GFP coding sequence is not in frame with the initiating ATG and therefore green colonies will be only obtained in recombinant clones, by restoration of the right frame resulting from the insertion of the synthetic gene. This vector allows regulating protein expression with IPTG but was found to be difficult for screening libraries on agar plates.
pETG derives from pTG, the insert has been cloned in pET17b (Novagen) and protein expression is regulated via the classical regulation of T7 RNA polymerase production system. This vector provides a better regulation of protein expression regulation than pTRAG. It can, be used in a strain which carries a chromosomal copy of the T7 RNA polymerase gene under control of the lacUV5 promoter and then the protein expression is control by addition of IPTG. More convenient for colony screening is to use this vector in a strain carrying the T7 polymerase gene under the control of the thermosensitive cI repressor (plasmid pGP1-2, (44)). Increasing temperature to 42°C after colony development allows producing the fusion protein and then to screen non delected genes.
PCocoG has been developed because with other vector, it remains some transcription leakage, (Novagen) which does not allow producing protein with pronounced toxic effects. It derived from pETcoco, has been designed for proteins that are suspected to be very toxic, owing to the additional possibility to induce the amplification of the vector copy number from a single copy to 20-50 copies per cell.

### Example 4 : Elimination of GFP tag.

Several methods are known to eliminate a tag sequence. Elimination can be performed by digestion with restriction enzymes at both sides of the tag and ligation. Introducing of a stop codon by site directed mutagenesis may be used to eliminate the C terminal peptide, in the more sophisticated vectors; this elimination is conditional by using a strain harboring the corresponding suppressor mutation. An usual technique is to introduce a sequence encoding a protease-cutting site, the tag is useful for protein purification and is withdrawn form the purified protein. The invention encompasses another technique to produce the protein without the tag : an Hind III site used to clone the gene is cut and filled using a polymerase, this filling and ligation step induces a frame shift replacing the first codon of GFP by a stop codon (Figure 4).

### References

1. Gupta, N. K., Ohtsuka, E., Sgaramella, V., Buchi, H., Kumar, A., Weber, H. & Khorana, H. G. (1968) Proc Natl Acad Sci U S A 60, 1338-44.
2. Crea, R., Kraszewski, A., Hirose, T. & Itakura, K. (1978) Proc Natl Acad Sci U S A 75, 5765-9.
3. Goeddel, D. V., Kleid, D. G., Bolivar, F., Heyneker, H. L., Yansura, D. G., Crea, R., Hirose, T., Kraszewski, A., Itakura, K. & Riggs, A. D. (1979) Proc Natl Acad Sci U S A 76, 106-10.
4. Sandhu, G. S., Aleff, R. A. & Kline, B. C. (1992) Biotechniques 12, 14-6.
5. Stemmer, W. P., Crameri, A., Ha, K. D., Brennan, T. M. & Heyneker, H. L. (1995) Gene 164, 49-53.
6. Young, L. & Dong, Q. (2004) Nucleic Acids Res 32, e59.
7. Scheibel, T. (2004) Microb Cell Fact 3, 14.
8. Feng, L., Chan, W. W., Roderick, S. L. & Cohen, D. E. (2000) Biochemistry 39, 15399-409.
9. Milek, R. L., Stunnenberg, H. G. & Konings, R. N. (2000) Vaccine 18, 1402-11.
10. Wheeler, V. C., Prodromou, C., Pearl, L. H., Williamson, R. & Coutelle, C. (1996) Gene 169, 251-5.
11. Hale, R. S. & Thompson, G. (1998) Protein Expr Purif 12, 185-8.
12. Baedeker, M. & Schulz, G. E. (1999) FEBS Lett 457, 57-60.
13. Withers-Martinez, C., Carpenter, E. P., Hackett, F., Ely, B., Sajid, M., Grainger, M. & Blackman, M. J. (1999) Protein Eng 12, 1113-20.
14. Gurkan, C. & Ellar, D. J. (2003) Biotechnol Appl Biochem 38, 25-33.
15. Kang, T. J., Kang, K. H., Kim, J. A., Kwon, T. H., Jang, Y. S. & Yang, M. S. (2004) Protein Expr Purif 38, 129-35.
16. Del Vecchio Blanco, F., Cafaro, V., Di Maro, A., Scognamiglio, R., Siniscalco, G., Parente, A. & Di Donato, A. (1998) FEBS Lett 437, 241-5.
17. Smith, H. 0., Hutchison, C. A., 3rd, Pfannkoch, C. & Venter, J. C. (2003) Proc Natl Acad Sci U S A 100, 15440-5.
18. Hoover, D. M. & Lubkowski, J. (2002) Nucleic Acids Res 30, e43.
19. Xiong, A. S., Yao, Q. H., Peng, R. H., Li, X., Fan, H. Q., Cheng, Z. M. & Li, Y. (2004) Nucleic Acids Res 32, e98.
20. Delroisse, J. M., Dannau, M., Gilsoul, J. J., El Mejdoub, T., Destain, J., Portetelle, D., Thonart, P., Haubruge, E. & Vandenbol, M. (2005) Protein Expr Purif 42, 286-94.
21. Subramanian, S., Kondaiah, P. & Radhakantha Adiga, P. (2002) Protein Expr Purif 26, 284-9.
22. Gurkan, C. & Ellar, D. J. (2003) Protein Expr Purif 29, 103-16.
23. Cherepanov, P., Pluymers, W., Claeys, A., Proost, P., De Clercq, E. & Debyser, Z. (2000) Faseb J 14, 1389-99.
24. Barber, M. J., Desai, S. K., Marohnic, C. C., Hernandez, H. H. & Pollock, V. V. (2002) Arch Biochem Biophys 402, 38-50.
25. Lin, Y., Cheng, G., Wang, X. & Clark, T. G. (2002) Gene 288, 85-94.
26. Carr, P. A., Park, J. S., Lee, Y. J., Yu, T., Zhang, S. & Jacobson, J. M. (2004) Nucleic Acids Res 32, e162.
27. Binkowski, B. F., Richmond, K. E., Kaysen, J., Sussman, M. R. & Belshaw, P. J. (2005) Nucleic Acids Res 33, e55.
28. Popovic, M., Coglievina, M., Guarnaccia, C., Verdone, G., Esposito, G., Pintar, A. & Pongor, S. (2006) Protein Exp. Purif., in press.
29. Hu, S., Li, L., Qiao, J., Guo, Y., Cheng, L. & Liu, J. (2006) Protein Expr Purif.
30. Lehmann, M., Kostrewa, D., Wyss, M., Brugger, R., D'Arcy, A., Pasamontes, L. & van Loon, A. P. (2000) Protein Eng 13, 49-57.
31. Kodumal, S. J., Patel, K. G., Reid, R., Menzella, H. G., Welch, M. & Santi, D. V. (2004) Proc Natl Acad Sci U S A 101, 15573-8.
32. Smith, J. & Modrich, P. (1997) Proc Natl Acad Sci U S A 94, 6847-50.
33. Dillon, P. J. & Rosen, C. A. (1990) Biotechniques 9, 298, 300.
34. Majumder, K. (1992) Gene 110, 89-94.
35. Di Donato, A., de Nigris, M., Russo, N., Di Biase, S. & D'Alessio, G. (1993) Anal Biochem 212, 291-3.
36. Au, L. C., Yang, F. Y., Yang, W. J., Lo, S. H. & Kao, C. F. (1998) Biochem Biophys Res Commun 248, 200-3.
37. Sommer, S. S., Groszbach, A. R. & Bottema, C. D. (1992) Biotechniques 12, 82-7.
38. de Boer, H. A., Comstock, L. J. & Vasser, M. (1983) Proc Natl Acad Sci U S A. 80,21-25.
39. Waldo, G. S., Standish, B. M., Berendzen, J. & Terwilliger, T. C. (1999) Nat Biotechnol 17, 691-5.
40. Maxwell, K. L., Mittermaier, A. K., Forman-Kay, J. D. & Davidson, A. R. (1999) Protein Sci 8, 1908-11.
41. Wigley, W. C., Stidham, R. D., Smith, N. M., Hunt, J. F. & Thomas, P. J. (2001) Nat Biotechnol 19, 131-6.
42. Ness, J. E., Kim, S., Gottman, A., Pak, R., Krebber, A., Borchert, T. V., Govindarajan, S., Mundorff, E. C. & Minshull, J. (2002) Nat Biotechnol 20, 1251-5.
43. Leung, D. W., Chen, E. & Goeddel, D. V. (1989) J. Methods Cell Molec. Biol. 1, 11-15.
44. Tabor, S. & Richardson, C. C. (1985) Proc Natl Acad Sci U S A 82, 1074-8.

## Claims

1. A method of producing mutated proteins and polypeptides comprising the steps consisting of:
a) inserting synthetic DNA fragments within a construct comprising a promoter allowing expression in micro-organisms and a reporter gene sequence;
b) transforming said micro-organisms with the construct obtained in step a);
c) culturing transformed micro-organisms of step b) and observing the expression of the reporter gene, wherein the expression of the reporter gene is indicative of expression of said synthetic DNA fragment, and wherein no expression of the reporter gene is indicative of frameshift and non-sense mutations, and
d) selecting transformed micro-organisms expressing the reporter protein,
e) optionally, testing the activity and/or properties of the protein or polypeptide expressed from synthetic DNA sequences selected in step d).

2. The method according to claim 1, wherein frameshift and non-sense mutations are eliminated by screening miro-organism colonnies expressing the reporter gene in step d).

3. The Method according to claim 1 or 2 which is applied to provide proteins and polypeptides with longer half-life, stability, increased productivity, enhanced solubility, or increased activity, modified selectivity, such as increasing or decreasing subtrate spectrum, or new functionnality.

4. The Method according to claim 1, which is applied to DNA manufacturers to provide customers with quality improved end-products.

5. The method according to one of claims 1 to 4, wherein the synthetic DNA sequence includes complete and partial genes or coding sequences or parts thereof, which sequences are synthesized by chemical reactions.

6. The method according to one of claims 1 to 5, wherein errors occuring during synthetic DNA fragment synthesis provide synthetic gene polymorphism.

7. The method according to one of claims 1 to 6, wherein step c) provides a library of mutants polypeptide or proteins for molecular evolution.

8. The method according to one of claims 1 to 7, wherein it is combined with multi-site directed mutagenesis.

9. The method according to one of claims 1 to 8, wherein the reporter proteins is selected from the group consiting of GFP (Green Fluorescence Protein), enhanced GFP (EGFP), YFP, BFP, RFP, as well as any variants thereof.

10. The method according to one of claims 1 to 8, wherein the reporter proteins is chosen from dectectable enzymes by fluorescence, phosphorescence, or histochimistry means, such as β-galactosidase, alcaline phosphatase, luciferase, or chloramphenicol-acetyl-transferase (CAT).

11. The method according to one of claims 1 to 10, wherein the reporter protein acts as a qualitative (folding and/or solubility) and semi-quantitative (expression rate of the protein) reporter.

12. The method according to one of claims 1 to 11, wherein step a) consists of introducing a synthetic DNA fragment ranging from 30 bases to 2000 bases, such as 300 bases, corresponding to a region of interest to mutate in the construct

13. The method according to one of claims 1 to 12, wherein the construct is an expression vector.

14. The method according to claim 13, wherein the synthetic DNA is amplified before cloning into the expression vector.

15. The method according to claim 14, wherein additional sequences are added at the extremities of synthetic sequences, said additional sequences comprising one or several restriction sites allowing cloning into said vector and sequences complementary to primers used for PCR amplification.

16. The method according to one of claims 1 to 15, wherein the micro-organism is eukaryote and prokaryote, such as E. Coli.

17. The use of a kit for performing the method according to one of claims 1 to 16 comprising at least one of the following components selected from :
- enzyme necessary for the phosphorylation,
- enzyme the ligation (ex: T4 DNA ligase),
- an expression vector with the reporter protein, and
- the competent cells E. Coli for the library formation.
